# NEW EUROPEAN PATENT SPECIFICATION

(11) **EP 0 921 812 B2**
(45) Date of publication and mention of the opposition decision: **21.12.2011**
(45) Mention of the grant of the patent: 07.11.2001
(21) Application number: 97928123.5
(22) Date of filing: 19.06.1997
(51) Int. Cl.: A61K 38/28, A61K 47/10

(54) **INSULIN PREPARATIONS CONTAINING A HALOGENIDE**
HALOGENID-ENTHALTENDE INSULINZUBEREITUNGEN
PREPARATIONS D'INSULINE CONTENANT DU HALOGENIDE

(30) Priority: 20.06.1996 DK 68596
(43) Date of publication of application: 16.06.1999
(73) Proprietor: NOVO NORDISK A/S, 2880 Bagsværd (DK)
(72) Inventor: NORUP, Elsebeth, DK-4040 Jyllinge (DK); LANGKJAER, Liselotte, DK-2930 Klampenborg (DK); HAVELUND, Svend, DK-2880 Bagsvaerd (DK)
(74) Representative: HOFFMANN EITLE
(86) International application number: PCT/DK1997/000268
(87) International publication number: WO 1997/048414

(56) References cited:
- WO-A1-95/00550
- US- - 4 472 385
- US- - 4 783 441
- US-A- 4 439 181
- DATABASE CAPLUS [Online] 11 July 1996 GUPTA V.: 'Effect of Solvent Additives on the Thermal Stability of Insulin', XP003001007 Retrieved from stn Database accession no. 1996:49528 & PERSPECTIVES ON PROTEIN ENGINEERING AND COMPLEMENTARY TECHNOLOGIES, COLLECTED PAPERS, INT'L SYMPOSIUM, 3RD 13 September 1994 - 17 September 1994, pages 209 - 212
- J. BRANGE AND L. LANGKAER ACTA PHARM. NORD. vol. 4, no. 3, 1992, pages 149 - 158
- KARL HEINZ WALLHÄUSSER: 'Praxis der Sterilisation Desinfektion - Konservierung', 1995 pages 425 - 426
- JENS BRANGE: 'Galenics of Insulin', 1987, SPRINGER-VERLAG
- PROTEIN ENGINEERING vol. 5, no. 6, 1992, pages 527 - 533

## Description

### Introduction

The present invention relates to parenteral pharmaceutical formulations consisting of an aqueous insulin preparation consisting of Asp^{B28} human insulin and further components as defined below, which preparation has superior chemical stability.

### Background of the invention

Diabetes is a general term for disorders in man having excessive urine excretion as in diabetes mellitus and diabetes insipidus. Diabetes mellitus is a metabolic disorder in which the ability to utilize glucose is more or less completely lost. About 2% of all people suffer from diabetes.

Since the introduction of insulin in the 1920's, continuous strides have been made to improve the treatment of diabetes mellitus. To help avoid extreme glycemia levels, diabetic patients often practice multiple injection therapy, whereby insulin is administered with each meal.

In the treatment of diabetes mellitus, many varieties of insulin preparations have been suggested and used, such as regular insulin, Semilente® insulin, isophane insulin, insulin zinc suspensions, protamine zinc insulin, and Ultralente® insulin. As diabetic patients are treated with insulin for several decades, there is a major need for safe and life-quality improving insulin preparations. Some of the commercially available insulin preparations are characterized by a fast onset of action and other preparations have a relatively slow onset but show a more or less prolonged action. Fast-acting insulin preparations are usually solutions of insulin, while retarded-acting insulin preparations can be suspensions containing insulin in crystalline and/or amorphous form precipitated by addition of zinc salts alone or by addition of protamine or by a combination of both. In addition, some patients are using preparations having both a fast onset of action and a more prolonged action. Such a preparation may be an insulin solution wherein protamine insulin crystals are suspended. Some patients do themselves prepare the final preparation by mixing an insulin solution with a suspension preparation in the ratio desired by the patient in question.

Human insulin consists of two polypeptide chains, the so-called A and B chains which contain 21 and 30 amino acids, respectively. The A and B chains are interconnected by two cystine disulphide bridges. Insulin from most other species has a similar construction, but may not contain the same amino acids at the positions corresponding in the chains as in human insulin.

The development of the process known as genetic engineering has made it possible easily to prepare a great variety of insulin compounds being analogous to human insulin. In these insulin analogues, one or more of the amino acids have been substituted with other amino acids which can be coded for by the nucleotide sequences. As human insulin, as explained above, contains 51 amino acid residues, it is obvious that a large number of insulin analogues is possible and, in fact, a great variety of analogues with interesting properties have been prepared. In human insulin solutions with a concentration of interest for injection preparations, the insulin molecule is present in associated form as a hexamer (Brange et al. Diabetes Care 13, (1990), 923 - 954). After subcutaneous injection, it is believed that the rate of absorption by the blood stream is dependent of the size of the molecule, and it has been found that insulin analogues with amino acid substitutions which counteract or inhibit this hexamer formation have an unusual fast onset of action (Brange et al.: Ibid). This is of great therapeutic value for the diabetic patient.

Pharmaceutical preparations which are based on analogues of human insulin have e.g. been presented by Heinemann et al., Lutterman et al. and Wiefels et al. at the "Frontiers in Insulin Pharmacology" International Symposium in Hamburg, 1992.

Furthermore, US 5 474 978 discloses a rapid acting parenteral formulation comprising a human insulin analogue hexamer complex consisting of six monomeric insulin analogues, zinc ions and at least three molecules of a phenolic derivative.

Normally, insulin preparations are administered by subcutaneous injection. What is important for the patient, is the action profile of the insulin preparation which is the action of insulin on the glucose metabolism as a function of the time from the injection. In this profile, inter alia, the time for the onset, the maximum value and the total duration of action are important. A variety of insulin preparations with different action profiles are desired and requested by the patients. One patient may, on the same day, use insulin preparations with very different action profiles. The action profile requested is, for example, depending on the time of the day and the amount and composition of any meal eaten by the patient.

Equally important for the patient is the chemical stability of the insulin preparations, especially due to the abundant use of pen-like injection devices such as devices which contain Penfill® cartridges, in which an insulin preparation is stored until the entire cartridge is empty. This may last for at least 1 to 2 weeks for devices containing 1.5-3.0 ml cartridges. During storage, covalent chemical changes in the insulin structure occur. This may lead to formation of molecules which are less active and potentially immunogenic such as deamidation products and higher molecular weight transformation products (dimers, polymers, etc.). A comprehensive study on the chemical stability of insulin is given in by Jens Brange in "Stability of Insulin", Kluwer Academic Publishers, 1994.

Acta Pharmaceutica Nordica 4(4), 1992, pp. 149-158 discloses insulin preparations in which the sodium chloride concentration has been varied in the range of 0 to 250 mM. However, the major part of the preparations, including all preparations which additionally comprise glycerol, contains a rather high amount of sodium chloride, i.e. 0.7% corresponding approximately to a concentration of 120 mM. It is stated in this document that whereas sodium chloride generally has a stabilizing effect on insulin preparations, glycerol and glucose lead to increased chemical deterioration.

Surprisingly, however, it has now been shown that insulin preparations of superior chemical stability can be obtained in the presence of glycerol and/or mannitol and rather low halogenide concentrations.

### Description of the invention

By "analogue of human insulin" as used herein is meant human insulin in which one or more amino acids have been deleted and/or replaced by other amino acids, including non-codeable amino acids, or human insulin comprising additional amino acids, i.e. more than 51 amino acids.

By "derivative of human insulin" as used herein is meant human insulin or an analogue thereof in which at least one organic substituent is bound to one or more of the amino acids.

In the present context the unit "U" corresponds to 6 nmol.

The present invention relates to a parenteral pharmaceutical formulation consisting of an aqueous insulin preparation consisting of :
Asp^{B28} human insulin,
glycerol and/or mannitol,
5 to 100 mM of a halogenide, and
further components as defined below.

The above insulin preparation has a high chemical stability which e.g. is reflected in a reduction in the formation of dimers and polymers and desamido insulins after storage. Furthermore, the physical stability is not deteriorated by the presence of the rather low amount of halogenide, and the insulin does not precipitate by long-term storage of the insulin preparations.

The halogenide is preferably an alkali or alkaline earth halogenide, more preferably a chloride such as sodium chloride.

Glycerol and/or mannitol is preferably present in an amount corresponding to a concentration of 100 to 250 mM, more preferably 140 to 250 mM, even more preferably 160 to 200 mM.

The insulin preparation according to the invention comprises a fast-acting analogue of human insulin, wherein position B28 is Asp, namely Asp^{B28} human insulin.

The insulin preparation preferably comprises 5 to 60 mM, more preferably 5 to 40 mM, of a halogenide.

The insulin preparation comprises 10 to 40 µg Zn/100 U insulin, preferably 10 to 26 µg Zn/100 U insulin.

In a preferred embodiment of the invention the insulin preparation comprises:
60 to 3000 nmol/ml, preferably 240 to 1200 nmol/ml, of the human insulin analogue,
10 to 40 µg Zn/100 U insulin, preferably 10 to 26 µg Zn/100 U insulin, and
0 to 5 mg/ml, preferably 0 to 4 mg/ml, of a phenolic compound.

As phenolic compounds, the preparation comprises a mixture of 0.5 to 4.0 mg/ml, preferably 0.6 to 4.0 mg/ml, of m-cresol and 0.5 to 4.0 mg/ml, preferably 1.4 to 4.0 mg/ml, of phenol, wherein the total content of phenolic compounds is not more than 5 mg/ml.

The insulin preparation of the present invention furthermore contains a phosphate buffer.

The invention is further illustrated by the following examples.

### EXAMPLE I

Insulin preparations containing dissolved Asp^{B28} human insulin with varying concentrations of sodium chloride was prepared in the following way:
370.4 mg Asp^{B28} human insulin was dissolved in water by adding 1.6 ml 0.2 N HCl and 49 µl zinc chloride solution (40 mg Zn/ml). 40 g of a solution containing 40 mg/ml glycerol, 3.75 mg/g phenol and 4.30 mg/g *m*-cresol was added to the insulin solution while mixing. 20 g of a solution containing a) 12.0 mg/g disodium phosphate dihydrate + 5 µl/g 2 N sodium hydroxide, b) 12.0 mg/g disodium phosphate dihydrate + 5 µl/g 2 N sodium hydroxide + 5 mg/g sodium chloride or c) 12.0 mg/g disodium phosphate dihydrate + 5 µl/g 2 N sodium hydroxide + 10 mg/g sodium chloride was added while mixing. pH was adjusted to pH 7.40 ± 0.05 and water added up to 100 ml. The Asp^{B28} Human insulin preparations were introduced into Penfill® cartridges and subjected to stability tests at 25 °C and 37 °C. The stability data obtained at the two different temperatures and at a phosphate concentration of 13.5 mM, 19.6 µg Zn/100 U insulin and pH = 7.4 are summarized in Table 1.

**TABLE 1**

| NaCl added (mM) | Total conc. Of Cl⁻ (mM) | Asp^{B28} Des-amido insulins formed (%) | Di- & polymers formed (%) |
|---|---|---|---|
| Data after 8 weeks at 37 °C | | | |
| 0 | 4.4 | 7.0 | 1.86 |
| 17 | 20.8 | 4.2 | 1.29 |
| 34 | 37.8 | 3.5 | 1.07 |

| Data after 8 months at 25 °C | | | |
|---|---|---|---|
| 0 | 4.4 | 6.4 | 1.0 |
| 17 | 20.8 | 4.1 | 0.8 |
| 34 | 37.8 | 3.7 | 0.8 |

### EXAMPLE II

Insulin preparations containing dissolved Asp^{B28} human insulin with varying concentrations of sodium chloride was prepared in the following way:
369.4 mg Asp^{B28} human insulin was dissolved in water by adding 1.6 ml 0.2 N HCl and 49 µl zinc chloride solution (40 mg Zn/ml). 40 g of a solution containing 40 mg/g glycerol, 3.75 mg/g phenol and 4.30 mg/g *m*-cresol was added to the solution while mixing. 10 g of a solution containing 24.0 mg/g disodium phosphate dihydrate and 11 µl/g 2 N sodium hydroxide was added while mixing. Finally varying amounts (0 g to 4.38 g) of a solution containing 40 mg/g sodium chloride were added while mixing up to a sodium chloride concentration mentioned in Table 4. pH was adjusted to 7.40 ± 0.05 and water added up to 100 ml. The Asp^{B28} Human insulin preparations were introduced into Penfill® cartridges and subjected to stability tests at 25 °C and 37 °C. The stability data obtained at the two different temperatures and at a phosphate concentration of 13.5 mM are summarized in Table 2.

**TABLE 2**

| NaCl added (mM) | Total conc. of Cl⁻ (mM) | Asp^{B28} Desamido insulins formed (%) | Di- & polymers formed (%) |
|---|---|---|---|
| Stability data after 6 weeks at 37 °C | | | |
| 5 | 8.5 | 4.1 | 0.99 |
| 12.5 | 16.3 | 3.6 | 0.92 |
| 20 | 23.8 | 3.0 | 0.87 |
| 25 | 28.8 | 3.0 | 0.82 |
| 30 | 33.8 | 2.8 | 0.80 |

| Stability data after 12 weeks at 25 °C | | | |
|---|---|---|---|
| 0 | 3.8 | 2.7 | 0.36 |
| 5 | 8.5 | 2.3 | 0.32 |
| 12.5 | 16.3 | 1.8 | 0.39 |
| 20 | 23.8 | 1.7 | 0.39 |
| 25 | 28.8 | 1.8 | 0.38 |
| 30 | 33.8 | 1.7 | 0.38 |

### EXAMPLE III

Insulin preparations containing dissolved Asp^{B28} human insulin with varying concentrations of phosphate and sodium chloride was prepared in the following way:
375.7 mg Asp^{B28} human insulin was dissolved in water by adding 1.6 ml 0.2 N HCl and 49 µl zinc chloride solution (40 mg Zn/ml). 20 g of a solution containing 80 mg/g glycerol, 7.50 mg/g phenol and 8.60 mg/g *m*-cresol was added to the solution while mixing. Varying amounts (3.71 g to 6.71 g) of a solution containing 24.0 mg/g disodium phosphate dihydrate and 11 µl/g 2 N sodium hydroxide was added while mixing, finally varying amounts (0 g to 3.65 g) of a solution containing 40 mg/g sodium chloride were added while mixing so as to obtain a sodium chloride concentration mentioned in table 6. pH was adjusted to pH 7.40 ± 0.05 and water added up to 100 ml. The Asp^{B28} Human insulin preparations were introduced into Penfill® cartridges and subjected to stability tests at 25 °C and 37 °C. The stability data at the two different temperatures and three different phosphate concentrations and at 19.6 µg Zn/100 U insulin and pH = 7.4 are summarized in Tables 3, 4 and 5.

**TABLE 3**

| NaCl added (mM) | Total conc. of Cl⁻ (mM) | Phosphate conc. (mM) | Asp^{B28} Des-amido insulins formed (%) | Di- & polymers formed (%) |
|---|---|---|---|---|
| Data after 6 weeks at 37 °C | | | | |
| 0 | 3.8 | 5 | 4.7 | 1.4 |
| 5 | 8.8 | 5 | 3.7 | 1.3 |
| 10 | 13.8 | 5 | 3.4 | 1.2 |
| 15 | 18.8 | 5 | 3.1 | 1.1 |
| 20 | 23.8 | 5 | 2.7 | 1.1 |
| 25 | 28.8 | 5 | 3.0 | 0.9 |

| Data after 12 weeks at 25 °C | | | | |
|---|---|---|---|---|
| 0 | 3.8 | 5 | 2.2 | 0.5 |
| 5 | 8.8 | 5 | 1.7 | 0.4 |
| 10 | 13.8 | 5 | 1.5 | 0.4 |
| 15 | 18.8 | 5 | 1.4 | 0.4 |
| 20 | 23.8 | 5 | 1.3 | 0.4 |
| 25 | 28.8 | 5 | 1.3 | 0.4 |

**TABLE 4**

| NaCl added (mM) | Total conc. of Cl⁻ (mM) | Phosphate conc. (mM) | Asp^{B28} Des-amido insulins formed (%) | Di- & polymers formed (%) |
|---|---|---|---|---|
| Data after 6 weeks at 37 °C | | | | |
| 0 | 3.8 | 7 | 4.3 | 1.2 |
| 5 | 8.8 | 7 | 3.6 | 1.2 |
| 10 | 13.8 | 7 | 3.1 | 1.1 |
| 15 | 18.8 | 7 | 3.1 | 1.0 |
| 20 | 23.8 | 7 | 2.9 | 1.0 |
| 25 | 28.8 | 7 | 2.8 | 1.1 |

| Data after 12 weeks at 25 °C | | | | |
|---|---|---|---|---|
| 0 | 3.8 | 7 | 2.0 | 0.5 |
| 5 | 8.8 | 7 | 1.7 | 0.4 |
| 10 | 13.8 | 7 | 1.4 | 0.4 |
| 15 | 18.8 | 7 | 1.5 | 0.4 |
| 20 | 23.8 | 7 | 1.4 | 0.4 |
| 25 | 28.8 | 7 | 1.3 | 0.4 |

**TABLE 5**

| NaCl added (mM) | Total conc. of Cl⁻ (mM) | Phosphate conc. (mM) | Asp^{B28} Des-amido insulins formed (%) | Di- & polymers formed(%) |
|---|---|---|---|---|
| Data after 6 weeks at 37 °C | | | | |
| 0 | 3.8 | 9 | 4.9 | 1.2 |
| 5 | 8.8 | 9 | 4.0 | 1.1 |
| 10 | 13.8 | 9 | 3.7 | 1.0 |
| 15 | 18.8 | 9 | 3.5 | 1.0 |
| 20 | 23.8 | 9 | 3.5 | 1.0 |
| 25 | 28.8 | 9 | 3.1 | 0.9 |

| Data after 12 weeks at 25 °C | | | | |
|---|---|---|---|---|
| 0 | 3.8 | 9 | n.d. | 0.4 |
| 5 | 8.8 | 9 | 1.8 | 0.4 |
| 10 | 13.8 | 9 | 1.5 | 0.4 |
| 15 | 18.8 | 9 | 1.5 | 0.4 |
| 20 | 23.8 | 9 | 1.6 | 0.4 |
| 25 | 28.8 | 9 | 1.4 | 0.4 |

## Claims

1. A parenteral pharmaceutical formulation consisting of an aqueous insulin preparation consisting of:
Asp^{B28} human insulin,
glycerol and/or mannitol,
5 to 100 mM of a halogenide,
a mixture of 0.5 to 4.0 mg/ml, preferably 0.6 to 4.0 mg/ml, of m-cresol and 0.5 to 4.0 mg/ml, preferably 1.4 to 4.0 mg/ml, of phenol as phenolic compounds, wherein the content of phenolic compounds is not more than 5 mg/ml, 10 to 40 µg Zn/100 U insulin, preferably 10 to 26 µg Zn/100 U insulin, and
a phosphate buffer.

2. A parenteral pharmaceutical formulation according to claim 1, wherein the halogenide is an alkali or alkaline earth halogenide, preferably a chloride, more preferably sodium chloride.

3. A parenteral pharmaceutical formulation according to claim 1 or 2, wherein the insulin preparation comprises: 100 to 250 mM, preferably 140 to 250 mM, more preferably 160 to 200 mM of glycerol and/or mannitol.

4. A parenteral pharmaceutical formulation according to any of the preceding claims, wherein the insulin preparation comprises 5 to 60 mM, preferably 5 to 40 mM, of a halogenide.

5. A parenteral pharmaceutical formulation according to any of the preceding claims, wherein the insulin preparation comprises:
60 to 3000 nmol/ml, preferably 240 to 1200 nmol/ml of Asp^{B28} human insulin.

## Patentansprüche

1. Parenterale pharmazeutische Formulierung, die aus einer wässrigen Insulinzubereitung besteht, die aus folgendem besteht:
Asp^{B28}-Humaninsulin,
Glycerin und/oder Mannit,
5 bis 100 mM eines Halogenids,
einer Mischung aus 0,5 bis 4,0 mg/ml, vorzugsweise 0,6 bis 4,0 mg/ml m-Kresol und 0,5 bis 4,0 mg/ml, vorzugsweise 1,4 bis 4,0 mg/ml Phenol als phenolische Verbindungen, worin der Gehalt der phenolischen Verbindungen nicht größer ist als 5 mg/ml,
10 bis 40 µg Zn/100 U Insulin, vorzugsweise 10 bis 26 µg Zn/100 U Insulin und
ein Phosphatpuffer.

2. Parenterale pharmazeutische Formulierung gemäß Anspruch 1, worin das Halogenid ein Alkali- oder Erdalkalihalogenid, vorzugsweise ein Chlorid und besonders bevorzugt Natriumchlorid ist.

3. Parenterale pharmazeutische Formulierung gemäß Anspruch 1 oder 2, worin die Insulinzubereitung folgendes umfasst:
100 bis 250 mM, vorzugsweise 140 bis 250 mM und besonders bevorzugt 160 bis 200 mM Glycerin und/oder Mannit.

4. Parenterale pharmazeutische Formulierung gemäß irgendeinem der vorangehenden Ansprüche, worin die Insulinzubereitung 5 bis 60 mM, vorzugsweise 5 bis 40 mM eines Halogenids umfasst.

5. Parenterale pharmazeutische Formulierung gemäß irgendeinem der vorangehenden Ansprüche, worin die Insulinzubereitung folgendes umfasst:
60 bis 3.000 nmol/ml, vorzugsweise 240 bi 1.200 nmol/ml Asp^{B28}-Humaninsulins.

## Revendications

1. Formulation pharmaceutique parentérale consistant en une préparation aqueuse d'insuline consistant en :
de l'insuline humaine Asp^{B28},
du glycérol et/ou du mannitol,
5 à 100 mM d'un halogénure,
un mélange de 0,5 à 4,0 mg/ml, de préférence 0,6 à 4,0 mg/ml, de m-crésol et de 0,5 à 4,0 mg/ml, de préférence 1,4 à 4,0 mg/ml, de phénol comme composés phénoliques, où la teneur en composés phénoliques n'est pas supérieure à 5 mg/ml,
10 à 40 µg de Zn/100 U d'insuline, de préférence 10 à 26 µg de Zn/100 U d'insuline, et
un tampon phosphate.

2. Formulation pharmaceutique parentérale selon la revendication 1 où l'halogénure est un halogénure alcalin ou alcalino-terreux, de préférence un chlorure, de préférence encore le chlorure de sodium.

3. Formulation pharmaceutique parentérale selon la revendication 1 ou 2 où la préparation d'insuline comprend : 100 à 250 mM, de préférence 140 à 250 mM, de préférence encore 160 à 200 mM de glycérol et/ou de mannitol.

4. Formulation pharmaceutique parentérale selon l'une quelconque des revendications précédentes où la préparation d'insuline comprend 5 à 60 mM, de préférence 5 à 40 mM, d'un halogénure.

5. Formulation pharmaceutique parentérale selon l'une quelconque des revendications précédentes où la préparation d'insuline comprend :
60 à 3000 nmol/ml, de préférence 240 à 1200 nmol/ml d'insuline humaine Asp^{B28}.
